# EUROPÄISCHE PATENTSCHRIFT

(11) **EP 3 594 721 B1**
(45) Veröffentlichungstag und Bekanntmachung des Hinweises auf die Patenterteilung: **15.02.2023**
(21) Anmeldenummer: 19183889.5
(22) Anmeldetag: 02.07.2019
(51) Int. Cl.: G01T 1/16, A61B 6/00, G01R 33/48

(54) **HYBRIDBILDGEBUNGSVORRICHTUNG**
HYBRID IMAGING DEVICE
DISPOSITIF D'IMAGERIE HYBRIDE

(30) Priorität: 09.07.2018 DE 102018211279
(43) Veröffentlichungstag der Anmeldung: 15.01.2020
(73) Patentinhaber: Bruker BioSpin MRI GmbH, 76275 Ettlingen (DE)
(72) Erfinder: Berneking, Arne, 76135 Karlsruhe (DE); Junge, Sven, 76275 Ettlingen (DE)
(74) Vertreter: Kohler Schmid Möbus Patentanwälte

(56) Entgegenhaltungen:
- DE-A1-102005 015 070
- DE-A1-102013 108 497
- US-A1- 2008 265 887
- US-A1- 2010 033 186

## Beschreibung

### Hintergrund der Erfindung

Die Erfindung betrifft eine Hybridbildgebungsvorrichtung nach Anspruch 1.

Eine derartige Hybridbildgebungsvorrichtung ist beispielsweise aus [13] und [16] bekannt.

### Hintergrund der Erfindung

Eine der größten Herausforderungen beim Entwerfen von MR-ET-Hybridscannern ist es, Interferenzen und gegenseitige Einflüsse von Komponenten beider Bildgebungsmodalitäten zu unterbinden. Bei MR-PET-Hybridscannern beispielsweise interagieren auf der einen Seite PET Komponenten mit den sensitiven Magnetfeldern des MRT, auf der anderen Seite beeinflussen die starken Magnetfelder der MRT-Anordnung, das statische *B*₀-Feld und die Gradientenfelder, die PET-Elektronik und damit die PET-Datenakquisition.

Zusätzlich kommt es zu elektromagnetischen Interferenzen. RF-Spulen werden auf die Larmorfrequenz abgestimmt und generieren ein magnetisches Feld *B*₁ im MHz-Bereich mit einer Amplitude im µT Bereich senkrecht zur *B*₀-Orientierung, um Spins anzuregen. Durch die hohe Frequenz kann die Erzeugung einer elektrischen Feldkomponente nicht verhindert werden. Die Leistung erreicht dabei den kW Bereich und stört die PET-Elektronik. Darüber hinaus sind RF-Spulen in der Lage, sehr kleine Signale von angeregten Spins zu detektieren, wobei ein Empfangssignal im µV Bereich liegen kann. Deswegen sind MRT-Spulen sehr rauschempfindlich und können selbst kleinste (störende) Signale detektieren, wenn diese im Bereich der Larmorfrequenz des MRT-Scanners liegen. Da sich in modernen MR-PET-Scannern eine Vielzahl an elektronischen Elementen befinden, unter anderem FPGA, ASIC und verschiedene Signalleitungen im MHz Bereich, sowie selbst die Digitalisierung der PET-Daten im MRT-Scanner mit breitbandigem Rauschen stattfinden kann, können abgesendete Signale der PET-Elektronik das Empfangssignal der RF-Spule stören oder komplett überlagern, so dass eine Detektion der MR-Signale unmöglich ist. Aus gleichem Grund wird eine MRT-Anordnung immer mittels geeigneter Maßnahmen z.B. mit einem Faraday-Käfig abgeschirmt.

Als Konsequenz werden für kombinierte MR- und PET-Systeme Abschirmungen der PET-Elektronik mit hohem Schirmungsfaktor benötig. Gleiches gilt, wenn man andere Elektronik als PET-Elektronik, beispielsweise SPECT(Einzelphotonen-Emissionscomputertomographie)-Elektronik, im geschirmten Untersuchungsraum installiert. Es wird daher immer eine zusätzliche Abschirmung der Elektronik dieser kombinierten Systeme im Untersuchungsraum innerhalb des Faraday-Käfigs benötigt.

Üblicherweise befindet sich deswegen eine Abschirmung gegen die elektromagnetischen Felder um die PET-Elektronik. Die Abschirmungsstärke hängt hierbei von der elektrischen Leitfähigkeit ab. Je höher die Leitfähigkeit, umso höher der Abschirmfaktor. Deswegen werden in der Regel Metalle oder Karbon als Schirmungsmaterial verwendet. Hierbei ist zu beachten, dass die Abschirmung nicht mit den Gradientenfeldern interagieren soll. Schaltet man die Gradientenspulen ein und aus, was während jeder MRT-Untersuchung geschieht, werden auf den Flächen der Abschirmung Wirbelströme induziert. Die induzierten Wirbelströme generieren selbst ein magnetisches Feld, was die Gradientenfelder superponiert und damit die Phasen- und Frequenzkodierung und somit letztendlich die Ortsauflösung der MRT-Bilder stört. Ebenfalls kann dies zu einer ungewollten Wärmeentwicklung durch diese Wirbelströme führen. Dies ist besonders kritisch für MRT-Sequenzen mit hohem Duty-Cycle wie EPI, EPIK oder Diffusionsbildgebung, bei denen Gradientenspulen schnellst möglich und häufig hintereinander mit hoher Gradientenamplitude geschaltet werden.

Neben der Abschirmung der PET-Elektronik ist bei bekannten MR-PET-Hybridscannern auch eine Abschirmung der RF-Sende- und Empfangsspule vorgesehen, um einen Einfluss der Umgebung auf die Resonanzkreise der RF-Spule zu unterbinden. Hier wird in der Regel ein geeigneter, geschlitzter Kupferschirm verwendet, welcher transparent für die Gradientenfelder ist und gleichzeitig wie ein geschlossener Leiter im Hochfrequenzbereich funktioniert.

Eine PET-Anordnung liegt in der Regel als PET-Ring vor, allerdings sind auch andere Geometrien möglich. Oftmals werden PET-Ringe in Kassettenform aufgebaut. Hierbei befinden sich die PET-Detektoren (in der Regel mit Szintillationskristallen), die -Ausleseelektronik und -Vorverarbeitung innerhalb einer Kassette. Ein Ring setzt sich dann aus einer Vielzahl von Kassetten zusammen. Hier ist es üblich einen Kupferschirm auf das Kassettengehäuse aufzubringen oder das Kassettengehäuse aus Carbon anzufertigen. Alternativ werden komplett elektrisch geschlossene PET-Ringe verwendet. Bei herkömmlichen Systemen wird als Abschirmung um den PET-Ring ein Schild installiert, der gegenseitige Einflüsse von PET-Elektronik und MR-HF-Spulen unterbindet. Darüber hinaus ist es bekannt, die Abschirmung durch Strukturierung und Aufbauen einer frequenzselektiven Abschirmung so zu optimieren, dass diese um die Larmorfrequenz maximal dämpft und gleichzeitig Gradientenfelder minimal beeinflusst [12]. Ein Konzept für derartige PET-Kassetten ist in [1] beschrieben

Die herkömmlichen Aufbauweisen benötigen einen RF-Spulenschirm für die RF-Spule und eine zusätzliche Abschirmung von der PET Elektronik. Hierfür wird entsprechend viel Platz in der Magnetröhre benötigt, welcher jedoch begrenzt und teuer ist. Außerdem benötigt der RF-Spulenschirm einen gewissen Abstand zur RF-Spule, da er wesentlichen Einfluss auf die Performance der Spule hat [2].

In der MRT und besonders der MR-PET Systementwicklung wird aus Kostengründen versucht, ein möglichst kompaktes Design zu erreichen. Je kleiner der Durchmesser des MR-Magneten ist, umso geringer ist der Preis für dieses MR-System. Außerdem ist es von Vorteil, sowohl die MRT-Empfangsspule als auch die PET-Detektoren so nahe wie möglich an das Untersuchungsobjekt zu bringen, um ein optimales Signal- zu Rauschverhältnis der Detektion der MR- und PET-Bildgebung zu erreichen und gleichzeitig PET-Detektoren einzusparen. Deshalb ist man interessiert, dass sich PET-Detektoren mit Elektronik und Schirmung mit voranschreitender Integration zunehmend näher am Untersuchungsobjekt befinden. Um störende Veränderungen der Gradientenfelder im Untersuchungsobjekt mit Bildstörungen und -Artefakten zu verhindern, müssen die Abschirmmaßnahmen geeignet sein, die Wirbelströme weitestgehend zu unterbinden.

Um ein solches kompaktes Design zu realisieren, wurde vorgeschlagen, den RF-Spulenschirm in die innere PET-Abschirmung zu integrieren (integrierte Abschirmung), vgl. [2]. Der zusätzliche geringe Platzgewinn kann hierbei verwendet werden, um den Abstand von integrierter Abschirmung und RF-Spule zu optimieren, so dass die RF-Spule eine bessere Performance zeigt. Auf diese integrierten Abschirmungen lassen sich ebenfalls gradienteneinflussreduzierende Methoden wie unter [1] beschrieben oder andere bekannte Methoden einsetzten.

Einen weiteren und deutlich höheren Platzgewinn erhält man, wenn man die integrierte Abschirmung hinter die Szintillatorkristalle der PET-Detektoren anordnet, da so der Abstand von RF-Spule zur integrierten Abschirmung von den Szintillatorkristallen besetzt werden kann. In [3], [4], [9] und [11] wird daher vorgeschlagen, die integrierte Abschirmung zwischen Szintillatorkristallen und Photosensoren anzuordnen.

Allerdings haben diese Anordnungen den Nachteil, dass die Schicht für die integrierte Abschirmung zwischen Szintillationskristall und Photosensor positioniert wird. Dadurch kommt es zu einer optischen Dämpfung und reduzierten Einkopplung von Photonen zwischen Szintillationskristall und Photosensor. Die Schicht sollte außerdem sehr dünn sein, da sonst noch mehr Photonen absorbiert werden. Dies hat zur Folge, dass die Abschirmstärke im Vergleich zu einem herkömmlichen Schirm erheblich reduziert wird. In [9] wird außerdem vorgeschlagen, ein Metallgrid zwischen SiPM Sensoren zu legen. Dieses ist wieder nur ein Abschirmgitter mit niedrigerer Abschirmstärke oder ein Schirmgitter mit geeigneter elektrischer Schirmwirkung und suboptimalen optischen Eigenschaften. Außerdem können die Photosensoren nicht beliebig dicht aneinandergereiht werden, was zwangsläufig zu Sensitivitätsverlusten durch die optische Dämpfung des PET-Ringes führt.

Ein Konzept, die Szintillatorkristalle aufzuteilen und in die Zwischenflächen Kupferflächen aufzubringen und diese zu verbinden, ist aus [6] und [10] bekannt. Hierbei ist jedoch nachteilig, dass die Abschirmung zwischen den Kristallen angeordnet ist und diese aufgeteilt werden müssen.

Ein weiteres Konzept ist aus [7] und [8] bekannt. Hier werden verschiedene Abschirmmaterialien zwischen die Szintillationskristalle eingefügt. Dieses Konzept eignet sich nur, wenn ein pixeliertes Szintillatorarray verwendet wird und das Gitter des Schirmes in der Größe der pixellierten Kristalle vorliegt. Es eignet sich nicht für monolithische Kristalle. Darüber hinaus ist die Abschirmstärke meist deutlich geringer, als die eines geschlossenen Schirmes. Des Weiteren muss hierbei die PET-Detektor-Geometrie an das Abschirmkonzept angepasst werden, sodass man z.B. nicht mehr frei in der Wahl des Pitchabstandes das Szintillatorarrays und der Länge der Szintillationskristalle ist.

offenbart ein kombiniertes Positron-Emissions-Tomographie- und Magnetresonanz-Tomographie-Gerät, bei dem eine Schirmhülle zwischen einer HF-Antenne und Szintillatorkristallen eines PET-Geräteteils vorgesehen ist. Die Schirmhülle befindet sich radial innerhalb eines Photosensors und kann mehrere Schichtanordnungen umfassen, wobei jede Schichtanordnung Leiterbahnen aufweist. Die Schichtanordnungen sind durch ein Dielektrikum voneinander getrennt.

offenbart ein PET/MRI Hybridsystem mit einer HF-Abschirmung und einer geteilten Gradientenspule.

offenbart eine Messeinheit für ein MR-System mit einem PET-Detektor und einer HF-Spulenanordnung, die eine integrale Einheit bilden. Zwischen einem Lichtdetektormodul und einem Detektorsockel ist eine HF-Abschirmung angeordnet.

offenbart ein kombiniertes Positronen-Emissions-Tomographie- (PET) und Magnetresonanztomographie- (MRT) System mit einer HF-Abschirmung, die in einem Detektorring zwischen den Szintillator- und Photodetektorkomponenten positioniert ist.

### Aufgabe der Erfindung

Es ist daher Aufgabe der Erfindung eine Hybridbildgebungsvorrichtung vorzuschlagen, die einerseits ein kompaktes Design aufweist und mit der andererseits ein optimiertes Signal- zu Rauschverhältnis der Detektion der MR- und ET-Bildgebung erreicht werden kann.

### Beschreibung der Erfindung

Diese Aufgabe wird erfindungsgemäß durch eine Hybridbildgebungsvorrichtung gemäß Patentanspruch 1 gelöst.

Bei der erfindungsgemäßen Hybridbildgebungsvorrichtung wird für die Abschirmung der ET-Elektronik gegen die RF-Spule und für die Abschirmung der RF-Spule gegen die ET-Elektronik eine gemeinsame integrierte innere Abschirmeinrichtung verwendet. Diese integrierte innere Abschirmeinrichtung ist zwischen RF-Resonatoranordnung und ET-Elektronik angeordnet, aber, im Gegensatz zu den aus dem Stand der Technik bekannten Abschirmeinrichtungen, nicht zwischen Photosensor und RF-Resonatorstruktur, sondern erfindungsgemäß radial außerhalb des Photosensors bzw. im Photosensor. "Radial" ist immer bezogen auf Richtungen senkrecht zur Längsachse der MRT-Anordnung, wobei "radial außerhalb" bedeutet: mit größerem Abstand zur z-Achse. Somit kann ein kompaktes Design realisiert werden, da, im Falle einer Detektoreinrichtung die Szintillationskristalle enthält, die Szintillationskristalle nahe am Untersuchungsobjekt positioniert werden können. Durch die erfindungsgemäße Positionierung der innere Abschirmeinrichtung radial außerhalb des Photosensors bzw. im Photosensor wird vermieden, dass zu detektierende Photonen durch die Abschirmung geblockt werden und nicht zum Photosensor gelangen. Hierdurch kann verhindert werden, dass die optischen Eigenschaften der Detektoreinrichtung beeinflusst werden. Es ergibt sich also eine verbesserte Performance der Hybridbildgebungsvorrichtung, da die optischen Eigenschaften der Detektoreinrichtung nicht verändert werden müssen. Als Detektoreinrichtung kann eine Anordnung mit Szintillationskristallen verwendet werden, in denen durch Szintillation niederenergetische Photonen (also Photonen im UV , nahinfraroten oder sichtbaren Wellenlängenbereich) erzeugt werden, die dann im Photosensor detektiert werden. Alternativ dazu kann als Photosensor auch ein Sensor zur Detektion von hochenergetischen Photonen (Gammastrahlung) verwendet werden, bspw. Gasdetektoren oder Halbleiterdetektor. In diesem Fall können die bei der Emissionstomografie entstehenden hochenergetischen Photonen detektiert werden. Im letzteren Fall werden in der erfindungsgemäßen Vorrichtung keine Szintillationskristalle benötigt.

Die Erfindung sieht vor, dass Durchkontaktierungen in der Abschirmeinrichtung vorhanden sind. Erfindungsgemäß ist entweder die innere in den Ausleseplatinen integriert, wobei elektrisch leitende Platinenschichten der Ausleseplatinen die innere Abschirmeinrichtung bilden oder Teil der innere Abschirmeinrichtung sind, oder die innere Abschirmeinrichtung ist in mindestens einer Sensorplatine der Platinenanordnung integriert ist, wobei die mindestens eine Sensorplatine mit Durchkontaktierungen für Signal- und Versorgungsleitungen versehen ist, oder die innere Abschirmeinrichtung ist im Photosensor integriert, wobei der Photosensor mit Durchkontaktierungen durch die im Photosensor integrierte innere Abschirmeinrichtung versehen ist, um die Ausgangssignale und Versorgungsspannungen hindurchzuführen. Die Durchkontaktierungen werden dazu genutzt, den Photosensor bzw. den sensitiven Teil des Photosensors, der immer radial innerhalb der inneren Abschirmeinrichtung (also außerhalb des abgeschirmten Bereichs) liegt, mit der ET-Elektronik zu verbinden und auszulesen. Insbesondere eignen sich hierzu Silizium-Durchkontaktierung (TSV) oder Glas-Durchkontaktierung (TGV). Mit TSV und TSG kann eine möglichst direkte Verbindung (also auf kürzestem Weg) realisiert werden. Damit können die Signalleitungslängen und wiederum die Bereiche, in denen Störsignal eingekoppelt werden können, reduziert werden. Darüber hinaus können bei dieser Ausführungsform die Signalleitungen der Photosensoren direkt unter den Photosensoren durch die Sensorplatine und der inneren Abschirmeinrichtung hindurchgeführt werden, so dass der Signalleitungsverlauf oberhalb der inneren Abschirmeinrichtung minimiert wird.

Die innere Abschirmeinrichtung weist vorzugsweise die gleiche Querschnittsform auf wie die ET-Anordnung, insbesondere einen ringförmigen oder quadratischen Querschnitt.

Das Material der inneren Abschirmeinrichtung ist nicht ferromagnetisch und umfasst vorzugsweise Kupfer, mit Silber ummanteltes Kupfer oder Silber.

Bei einer besonders bevorzugten Ausführungsform der erfindungsgemäßen Hybridbildgebungsvorrichtung ist die Emissions-Tomographie-Anordnung eine Positronen Emissions-Tomographie (PET)-Anordnung, wobei die Detektoreinrichtung eine Mehrzahl von (vorzugsweise kreisförmig angeordneten) Photosensoren umfasst. Alternativ hierzu kann als Emissions-Tomographie-Anordnung eine SPECT-Anordnung verwendet werden.

Die innere Abschirmeinrichtung soll eine ausreichend hohe Kapazität aufweisen, so dass für die innere Abschirmeinrichtung ein Widerstand kleiner 1Ω im MHz-Bereich erreicht wird und gleichzeitig ein Widerstand von >1 kΩ im kHz-Bereich. Die Einstellung der Höhe der Kapazität wird vorzugsweise mittels überlappender Flächen aus elektrisch leitfähigen Material realisiert. Bei einer besonders bevorzugten Ausführungsform ist daher vorgesehen, dass die innere Abschirmeinrichtung mehrere, sich vorzugsweise überlappende Abschirmflächen aus elektrisch leitfähigem Material umfasst. Im Überlappungsbereich ist es erforderlich, dass sich ein Isolator, der vorzugsweise ein Dielektrikum ist, zwischen den Abschirmflächen befindet.

Eine besonders einfache Implementierung und Realisierung einer frequenzselektiven Abschirmung kann dadurch realisiert werden, dass die Abschirmflächen in mindestens einer Platine der Platinenanordnung integriert oder auf mindestens einer Platine der Platinenanordnung aufgebracht sind, wobei es sich bei der mindestens einen Platine um Ausleseplatine(n) und/oder Sensorplatine(n) der ET-Anordnung handelt. Die Ausleseplatine umfasst die ET-Elektronik und gegebenenfalls den Photosensor. Der Photosensor kann jedoch auch auf einer eigenen Sensorplatine aufgebracht sein. Vorzugsweise sind die Abschirmflächen, welche die innere Abschirmeinrichtung bilden, in und/oder auf derselben Platine der Platinenanordnung (also in/auf der Sensorplatine oder in/auf der Ausleseplatine) angeordnet.

Die Ausleseplatinen und gegebenenfalls die optionalen Sensorplatinen sind radial außerhalb oder auf derselben radialen Position wie die innere Abschirmeinrichtung angeordnet.

Vorzugsweise umfasst die innere Abschirmeinrichtung mindestens eine elektrisch leitfähige Platinenschicht (Schicht der Sensorplatinen und/oder Ausleseplatinen). Alternativ oder zusätzlich kann auf die Platinen auch mindestens eine zusätzliche elektrisch leitfähige Schicht als Abschirmfläche aufgebracht werden. Durch die erfindungsgemäße Integration der innere Abschirmeinrichtung in die Platinenanordnung oder dem Sensorelement kann auf ein zusätzliches Gehäuse für die ET-Anordnung, auf das z. B. eine Kupferschicht zur Abschirmung aufgebracht werden muss, verzichtet werden.

Vorzugsweise ist die Platinenanordnung mehrlagig und umfasst zumindest teilweise mehrere Abschirmflächen, wobei die verschiedenen Abschirmflächen innerhalb der Platinenanordnung auf mehrere Lagen der Platinenanordnung verteilt sind. Es wird also eine Abschirmung über mehrere Platinenlagen entworfen. Durch die Verwendung mehrerer Lagen für die innere Abschirmeinrichtung lässt sich die Abschirmstärke deutlich erhöhen. Alternativ hierzu können die Abschirmschichten, welche gewöhnlich eine Dicke der 10-fachen Skintiefe der entsprechenden Larmorfrequenz haben sollten, dünner gestaltet werden. Es wurde in [5] gezeigt, dass das Aufteilen der Schichtdicke in mehrere Schichten die Abschirmstärke nicht wesentlich verringert. Bei Verwendung mehrerer dünner Schichten reduzieren sich jedoch die Gradientenwirbelströme im Vergleich zu einer dickeren Schicht.

Bei einer bevorzugten Ausführungsform sind die Abschirmflächen innerhalb einer der Platine der Platinenanordnung kapazitiv miteinander verbunden. Die Kapazitäten sind dabei vorzugsweise so gewählt, dass ein möglichst niederohmiger Wiederstand im Hochfrequenzbereich im Bereich der Larmorfrequenz (1 MHz bis 10 GHz abhängig von der Feldstärke B₀) und ein möglichst hoher Widerstand im Niederfrequenzbereich (kHz) entsteht. Die kapazitive Verbindung der Abschirmflächen innerhalb einer Platine kann durch auf der entsprechenden Platine aufgelötete Kondensatoren realisiert sein. Die Kondensatoren können z.B. auf der Rückseite der Sensorplatinen oder auf der Vorder- oder Rückseite der Ausleseplatine aufgelötet sein. Befinden sich die Abschirmschichten nicht auf der gleichen Seite der Platine, auf der sich die Kondensatoren befinden, kann eine Kontaktierung mit Via-Kontaktierungen durchgeführt werden.

Anstelle von eingelöteten Kondensatoren lassen sich die Kapazitäten auch mit Hilfe von zwei oder mehreren Abschirmlagen innerhalb der Platinenanordnung realisieren. Bei einer besonders bevorzugten Ausführungsform ist daher die kapazitive Verbindung der Abschirmflächen innerhalb der Platinenanordnung durch Überlagerung der Abschirmflächen innerhalb der Platinenanordnung, insbesondere innerhalb einer Platine der Platinenanordnung, realisiert. Je nachdem, welche Kapazität gewünscht ist, kann der Überlappungsgrad der Abschirmflächen festgelegt werden gemäß der Formel C=ε₀εᵣA/d. Durch die Wahl des Dielektrikums und somit der Dielektrizitätskonstante εᵣ, der Größe der Überlappungsflächen und des Abstands der Überlappungsflächen lässt sich eine Kapazität einstellen, die frequenzabhängig unterschiedliche hohe Impedanzen und frequenzabhängige Dämpfungen darstellen.

Vorzugsweise bildet die innere Abschirmeinrichtung eine geschlossenen RF-Schirmfläche. Unter einer "geschlossenen RF-Schirmfläche" versteht man eine Abschirmung die im hochfrequenten Bereich (1 MHz bis 10 GHz) eine für elektromagnetische Wellen in sich geschlossene und an allen Stellen der Abschirmeinrichtung undurchlässige Abschirmung erzeugt. Die Platinen können dazu mittels eines leitfähigen Materials aneinander gelötet sein oder durch Press- oder Steckverbindungen in elektrischen Kontakt gebracht werden, wodurch jedoch bspw. durch Schalten der Gradientenspulen Wirbelströme induziert werden können.

Weiterhin kann die geschlossene RF-Schirmfläche dadurch realisiert sein, dass die innere Abschirmeinrichtung integrierte Kapazitäten zwischen den einzelnen Abschirmflächen aufweist. Dadurch wird eine kapazitive Kopplung der Abschirmflächen erreicht, und die RF-Schirmfläche lässt sich auf mehrere Platinen der Platinenanordnung ausbreiten, und bildet so eine geschlossenen RF-Schirmfläche. Eine Möglichkeit, dies zu realisieren, ist, die Abschirmflächen verschiedener Platinen mittels eingelöteter Kondensatoren zu verbinden oder durch eine Überlappung der Abschirmflächen analog einem Plattenkondensator. Die Kapazitäten der Kondensatoren bzw. der kapazitiven Kopplung sind so gewählt, dass im RF-Bereich eine niedrige Impedanz gegeben ist, die innere Abschirmeinrichtung im Bereich der Larmorfrequenz also geeignet leitfähig ist, und im kHz Bereich der Gradienten eine hohe Impedanz vorliegt. Dies garantiert eine hohe Abschirmung des RF-Signals im Bereich der Larmorfrequenz der MRT-Anordnung. Zusätzlich werden die Flächen für induzierte Gradientenströme (Wirbelströme) verkleinert und somit Wirbelstromstärken reduziert.

Vorzugsweise ist zusätzlich eine äußere Abschirmeinrichtung vorhanden, die radial außerhalb der Detektoreinrichtung und der Platinenanordnung angeordnet ist. Die äußere Abschirmeinrichtung schirmt die ET-Elektronik in radial innere Richtung ab. Um einen vollständig geschlossenen (also einen die ET-Elektronik allseitig vollständig umschließenden) Schirm zu bilden, sind die innere Abschirmeinrichtung und die äußere Abschirmeinrichtung vorzugsweise an den Rändern (axiale Enden) miteinander elektrisch verbunden bzw. elektromagnetisch, insbesondere kapazitiv oder induktiv gekoppelt.

Bei einer besonders bevorzugten Ausführungsform sind Komponenten der ET-Elektronik radial zwischen innerer Abschirmeinrichtung und äußerer Abschirmeinrichtung angeordnet. Wenn die innerer Abschirmeinrichtung in die Ausleseplatinen, welche die ET-Elektronik beinhalten, integriert ist, sind also die Komponenten der ET-Elektronik auf der der äußeren Abschirmeinrichtung zugewandten Seite der Ausleseplatinen angeordnet. Vorzugsweise sind Signal- und Versorgungsleitungen der ET-Elektronik zwischen innerer Abschirmeinrichtung und äußerer Abschirmeinrichtung angeordnet. Somit kann die ET-Elektronik optimal abgeschirmt werden. Um Einkopplungen von Störsignalen in durch die geschlossene RF-Schirmfläche zu unterbinden, kann die Hybridbildgebungsvorrichtung RF-Filter umfassen.

Gemäß der Erfindung ist jedoch vorgesehen, dass RF-Filter direkt in der inneren Abschirmeinrichtung oder in der Platinenanordnung insbesondere innerhalb einer Platine integriert sind.

Ist die innerer Abschirmeinrichtung direkt in der Detektoreinrichtung integriert, sind die RF-Filter vorzugsweise in den Photosensoren selbst oder in den Signalausgang der Photosensoren integriert. Dadurch kann die Effizienz der Abschirmung noch weiter erhöht werden.

Als Filter können dem Fachmann bekannte Filtertypen gewählt werden, so dass Signale im MHz- bis GHz-Bereich (entsprechend der Larmorfrequenz) geblockt werden. Diese Filter können beispielsweise (aber nicht einschränkend) Tiefpass-, Bandpass- und Hochpassfilter sein, insbesondere Tiefpassfilter 3. Ordnung und Notchfilter.

Vorzugsweise handelt es sich bei der Detektoreinrichtung um einen Silizium-Photomultiplier (SiPM). Grundsätzlich kann das erfindungsgemäße Abschirmungsprinzip bei unterschiedlichen Detektoreinrichtungen eingesetzt werden, jedoch ist die Verwendung von SiPM bevorzugt, da diese auf Grund ihrer hohen intrinsischen Verstärkung gegen HF-Einstrahlung weniger sensibel sind. Das Ausgangssignal ist dann weniger störanfällig.

Als Platinen, in denen die innere Abschirmeinrichtung integriert ist, können neben dem Fachmann bekannten Platinenmaterialien beispielsweise Glasplatinen verwendet werden. Die Abschirmschichten können in den Glasplatinen in Form von Kupferschichten integriert sein. Dadurch erhält man eine zusätzliche Platzersparnis, da sich die RF-Filter miniaturisiert und kleiner als bei herkömmlichen Platinen direkt als Kupferstruktur in die Glasplatine integrieren lassen.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

- Fig. 1: zeigt einen Querschnitt der radial innerhalb des Gradientenspulensystems angeordneten Komponenten einer MR-PET Hybridbildgebungsvorrichtung mit getrennten innere Abschirmeinrichtungen für die PET-Elektronik und die RF-Resonatorstruktur gemäß dem Stand der Technik.
- Fig. 2: zeigt einen Querschnitt der radial innerhalb des Gradientenspulensystems angeordneten Komponenten einer MR-PET Hybridbildgebungsvorrichtung mit einer integrierten RF/PET-Abschirmeinrichtung zwischen Szintillatorkristallen und Photosensoren gemäß dem Stand der Technik.
- Fig. 3: zeigt einen Querschnitt einer erfindungsgemäßen MR-PET-Hybridbildgebungsvorrichtung.
- Fig. 4: zeigt den Aufbau einer Hälfte einer MR-PET-Hybridbildgebungsvorrichtung (Längsschnitt).
- Fig. 5: zeigt einen Ausschnitt einer ersten Ausführungsform des erfindungsgemäßen Abschirmkonzepts mit innere Abschirmeinrichtung im Photosensor (Längsschnitt).
- Fig. 6: zeigt einen Ausschnitt einer zweiten Ausführungsform des erfindungsgemäßen Abschirmkonzepts mit innere Abschirmeinrichtung in der Sensorplatine (Längsschnitt).
- Fig. 7: zeigt einen Ausschnitt einer dritten Ausführungsform des erfindungsgemäßen Abschirmkonzepts mit innere Abschirmeinrichtung in der Ausleseplatine (Längsschnitt).
- Fig. 8: zeigt eine Aufsicht eines Ausschnitts einer innere Abschirmeinrichtung mit mehreren mittels Kondensatoren kapazitiv gekoppelten Abschirmflächen (Abwicklungsdarstellung).
- Fig. 9: zeigt eine Schnittdarstellung eines Ausschnitts einer innere Abschirmeinrichtung mit überlappenden Abschirmflächen (Längsschnitt).

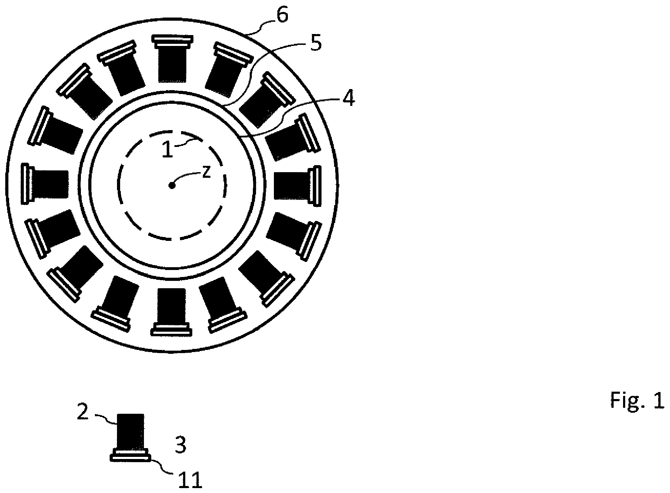

**Fig. 1** zeigt einen herkömmlichen Aufbau einer Hybridbildgebungsvorrichtung (hier: MR-PET-Scanner). Hierbei befindet sich eine RF-Resonatorstruktur **1** im Zentrum der Vorrichtung. Radial außerhalb der RF-Resonatorstruktur 1 ist eine PET-Anordnung mit Szintillatorkristallen **2** und einem Photosensor **3** (mit mehreren Sensorelementen) zur Detektion von in den Szintillatorkristallen 2 erzeugten Photonen sowie mit einer Ausleseplatine **11** mit PET-Elektronik angeordnet. Radial zwischen der RF-Resonatorstruktur 1 und der PET-Anordnung ist ein RF-Spulenschirm **4** angeordnet, der verhindert, dass die RF-Resonatorstruktur 1 mit der Umgebung im Nahfeldbereich interagiert und dadurch verstimmt wird. Darüber hinaus ist die PET-Anordnung durch eine innere PET-Abschirmung **5** und eine äußere PET-Abschirmung **6** vor elektromagnetischer Interaktion im MHz bis GHz-Bereich geschützt.

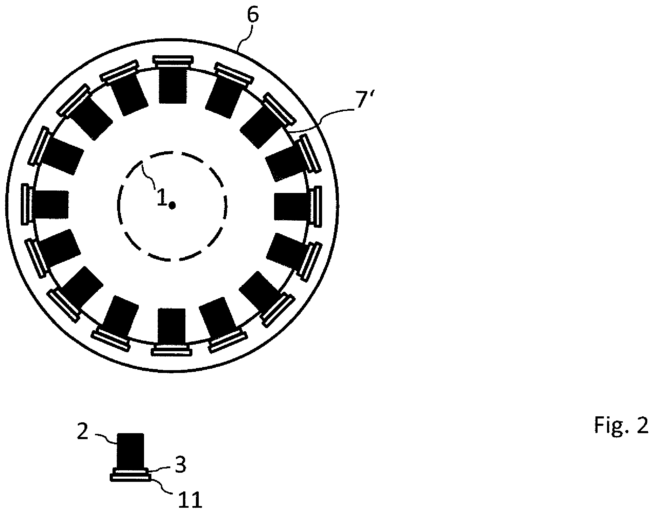

**Fig. 2** zeigt einen aus dem Stand der Technik bekannten MR-PET Scanner mit einer integrierten innere Abschirmeinrichtung **7'**, welche die Funktionen der inneren PET-Abschirmung 5 und des RF-Spulenschirms 4 aus der in Fig. 1 gezeigten Vorrichtung übernimmt. Die integrierte innere Abschirmeinrichtung **7'** befindet sich innerhalb der PET-Anordnung zwischen Szintillatorkristallen 2 und Photosensor 3, sodass diese zusammen mit der äußeren PET-Abschirmung **6** die Photosensoren 3 und die PET-Elektronik von RF-Strahlung der RF-Resonatorstruktur 1 schützt und die RF-Resonatorstruktur 1 durch die integrierte innere Abschirmeinrichtung **7'** von Störeinflüssen der PET-Elektronik der PET-Anordnung abgeschirmt wird.

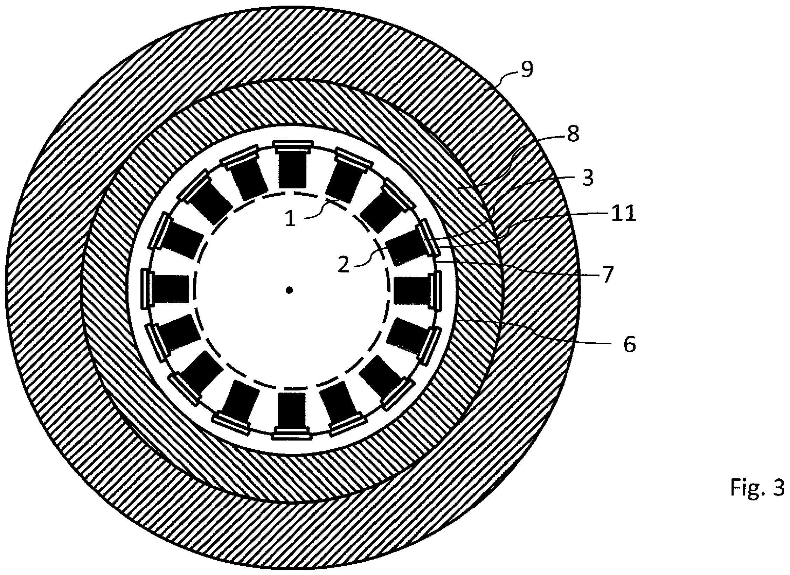

**Fig. 3** zeigt eine Hybridbildgebungsvorrichtung gemäß der vorliegenden Erfindung mit MR-Anordnung und PET-Anordnung. Die MR-Anordnung umfasst neben der RF-Resonatorstruktur 1 ein Gradientenspulensystem **8** mit einer Längsachse **z** und eine Magnetspulenanordnung **9** zur Erzeugung eines statischen Magnetfeldes. Die PET-Anordnung mit Szintillatorkristallen 2, Photosensor 3 und Ausleseplatine 11 ist radial zwischen dem Gradientenspulensystem 8 und der RF-Resonatorstruktur 1 angeordnet. Die Sensorelemente des Photosensors 3 sind koaxial (mit der Längsachse z des Gradientenspulensystems 8 als gemeinsame Achse), insbesondere konzentrisch, zum Gradientenspulensystem angeordnet. Im vorliegenden Beispiel weist der Photosensor 3 im Querschnitt insgesamt eine kreisförmigen Anordnung der Sensorelemente auf. Es sind jedoch auch andere Geometrien möglich, bspw. eine Anordnung der Sensorelemente entlang eines quadratischen Querschnitts oder eine im Querschnitt lineare Anordnung der Sensorelemente auf zwei gegenüberliegenden Abschnitten.

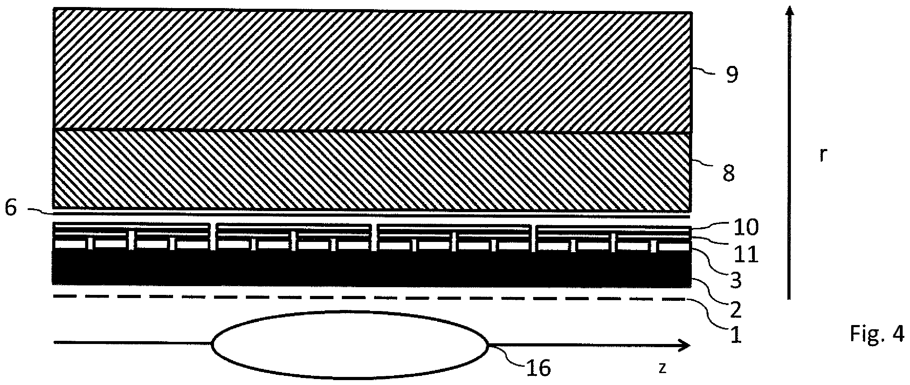

Auch hier ist eine äußere Abschirmeinrichtung 6 sowie eine integrierte innere Abschirmeinrichtung 7 (also eine kombinierte PET/RF-Abschirmeinrichtung) vorgesehen. Erfindungsgemäß ist diese innere Abschirmeinrichtung 7 radial außerhalb des Photosensors 3 angeordnet oder im Photosensor 3 integriert. Die Szintillationskristalle 2 können somit den Abstand zwischen RF-Resonatorstruktur 1 und integrierter Abschirmung 7 einnehmen und daher nahe an der RF-Resonatorstruktur 1 angeordnet werden, wie in Fig. 3 gezeigt. Durch die erfindungsgemäße Anordnung der innere Abschirmeinrichtung 7 im oder radial außerhalb des Photosensors 3 können die in den Szintillatorkristallen 2 erzeugten optischen Photonen ungehindert zum Photosensor 3 gelangen, wodurch die Performance der Hybridbildgebungsvorrichtung gegenüber der in Fig. 2 gezeigten Vorrichtung verbessert wird. **Fig. 4** zeigt den detaillierten Aufbau einer MR/PET-Hybridbildgebungsvorrichtung (integrierte innere Abschirmeinrichtung ist nicht dargestellt), wobei nach oben die radiale Richtung r und nach rechts die axiale Richtung der Längsachse z aufgetragen ist. Der Photosensor 3 ist radial außerhalb, an den Szintillatorkristallen 2 angrenzend angeordnet, gefolgt von Platinen (Sensorplatinen **10** und Ausleseplatinen **11** mit PET-Elektronik). Die von einem radial innerhalb der RF-Resonatorstruktur 1 im Zentrum der Vorrichtung angeordneten Untersuchungsobjekt **16** ausgehenden hochenergetischen Photonen (Gammastrahlung) gelangen in die Szintillatorkristalle 2, wo optische Photonen (also Photonen im UV oder sichtbaren Wellenlängenbereich) erzeugt werden, die mittels des wiederum radial weiter außen angeordneten Photosensors 3 detektiert werden. Die äußerste Abschirmeinrichtung 6 ist radial zwischen den Platinen 10, 11 und dem Gradientenspulensystem 8 angeordnet.

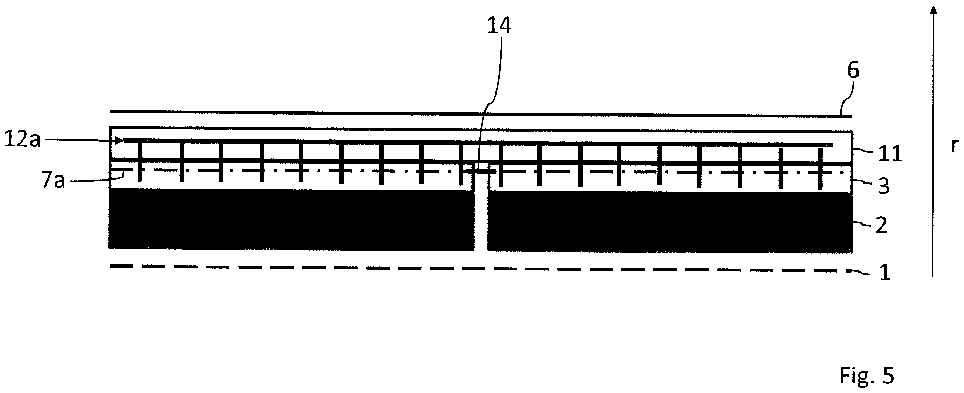

**Fig. 5** zeigt die Anordnung einer integrierten innere Abschirmeinrichtung 7a (gestrichpunktet dargestellt) für eine erste Variante der erfindungsgemäßen Hybridbildgebungsvorrichtung. Die innere Abschirmeinrichtung 7a ist hier im Photosensor 3 integriert. Um eine ungehinderte Übertragung der im Photosensor 3 generierten Photoelektronen durch die innere Abschirmeinrichtung 7a hindurch in die darüber liegenden Platinen (hier: Ausleseplatinen 11) zu gewährleisten, sind im Photosensor 3 Durchkontaktierungen **12a** vorgesehen.

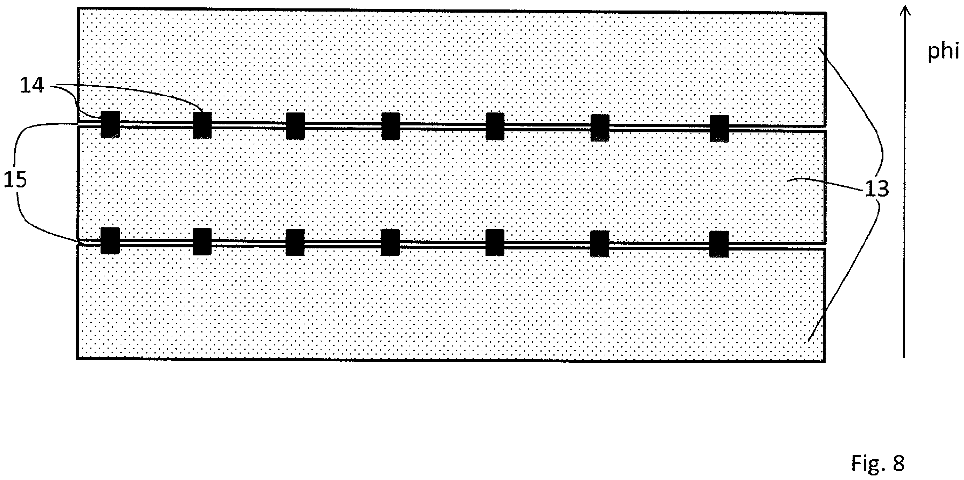

Die innere Abschirmeinrichtung 7a ist über mehrere Sensorelemente des Photosensors 3 verteilt und umfasst mehrere Abschirmflächen **13**, die mittels Abschirmverbindungen **14** miteinander verbunden sind (s. auch Fig. 8).

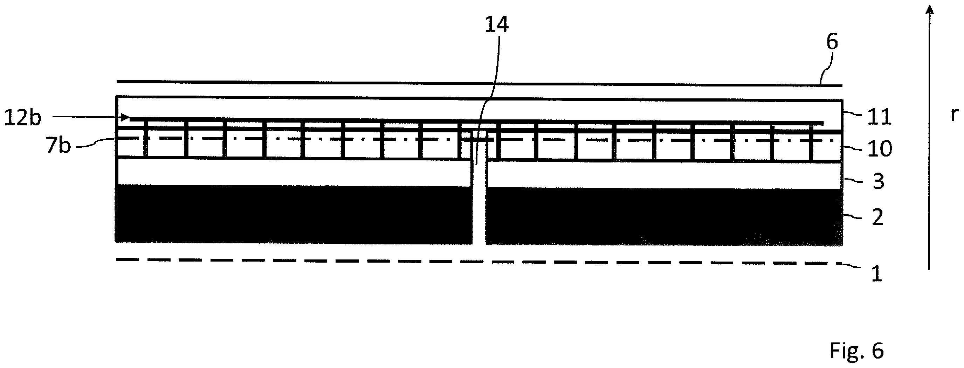

Bei einer zweiten Variante, die in **Fig. 6** gezeigt ist, ist eine integrierte innere Abschirmeinrichtung **7b** (gestrichpunktet dargestellt) in den Sensorplatinen 10 integriert, wobei elektrisch leitende Platinenschichten der Sensorplatinen 10 die innere Abschirmeinrichtung 7b bilden oder Teil der innere Abschirmeinrichtung 7b sind. Mittels Durchkontaktierungen **12b** in den Sensorplatinen 10 wird der Photosensor 3 mit den radial außerhalb der innere Abschirmeinrichtung 7b angeordneten Ausleseplatinen 11 verbunden.

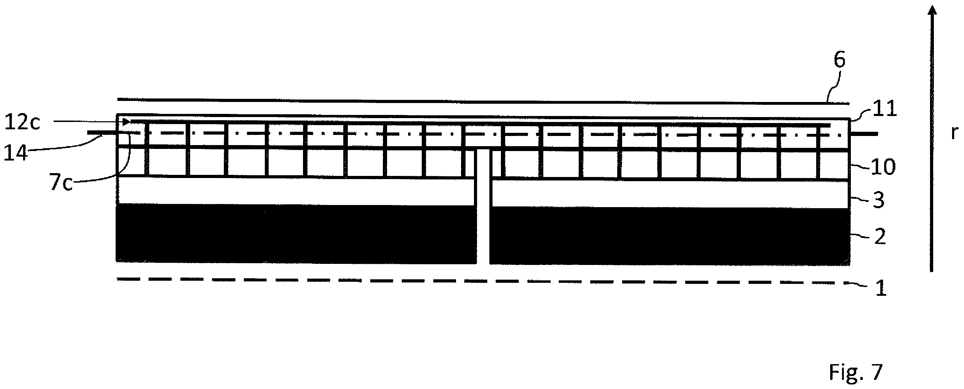

Bei einer dritten Variante, die in **Fig. 7** gezeigt ist, ist eine integrierte innere Abschirmeinrichtung **7c** (gestrichpunktet dargestellt) in den Ausleseplatinen 11 integriert, wobei elektrisch leitende Platinenschichten der Ausleseplatinen 11 die innere Abschirmeinrichtung 7c bilden oder Teil der innere Abschirmeinrichtung 7c sind. Mittels Durchkontaktierungen **12c** in den Sensorplatinen 10 und den Ausleseplatinen 11 wird der Photosensor 3 mit den radial außerhalb der innere Abschirmeinrichtung 7b angeordneten Komponenten der Ausleseplatinen 11 verbunden.

Die erfindungsgemäße innere Abschirmeinrichtung 7, 7a, 7b, 7c umfasst mehrere Abschirmflächen **13**, die kapazitiv miteinander gekoppelt sind. Dies kann bspw. mittels Kondensatoren **14** erfolgen, wie in **Fig. 8** in einer Abwicklungsdarstellung gezeigt. Die Abschirmflächen 13 sind hier in Umfangsrichtung **phi** nebeneinander angeordnet und durch Schlitze **15** voneinander getrennt, die von den Kondensatoren 14 überbrückt werden.

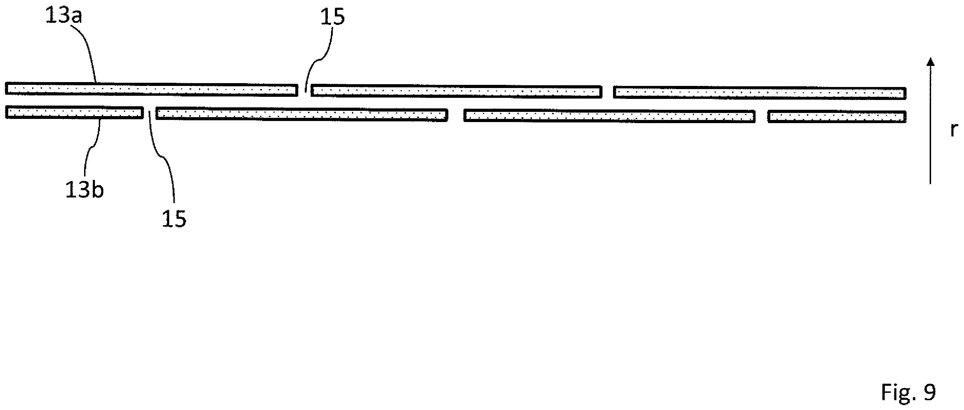

**Fig. 9** zeigt eine weitere Möglichkeit der kapazitiven Kopplung von Abschirmflächen **13a, 13b** in Schnittdarstellung senkrecht zur axialen Richtung z der Vorrichtung. In der in Fig. 9 gezeigten Ausführungsform sind die Abschirmflächen 13a, 13b in zwei radialen voneinander beabstandeten Schichten angeordnet, wobei sich die Abschirmflächen 13a, 13b in Umfangsrichtung u überlappen, wodurch die gewünschte kapazitive Kopplung erreicht wird.

Durch die in Fig. 8 und Fig. 9 gezeigten kapazitiven Kopplungen der Abschirmflächen wird eine geschlossene RF-Schirmfläche erzeugt. Diese kann zusammen mit der äußeren Abschirmeinrichtung eine geschlossene Abschirmung für die ET-Elektronik bilden, indem die integrierte innere Abschirmeinrichtung mit der äußeren Abschirmeinrichtung durch weitere RF-Schirmflächen (nicht gezeigt) verbunden wird.

Erfindungsgemäß wird eine integrierten EP/RF-Abschirmeinrichtung hinter dem (radial außerhalb) bzw. im Photosensor positioniert, um sicherzustellen, dass eine groß Anzahl der in den Szintillatorkristallen erzeugten Photonen detektiert wird. Auf diese Weise wird eine kompakte Hybridbildgebungsvorrichtung mit einer optimierten Performance preiswerter realisiert.

### Bezugszeichenliste

- 1: RF-Resonatorstruktur
- 2: Szintillatorkristalle
- 3: Photosensoren
- 4: RF-Spulenschirm
- 5: innere PET-Abschirmung
- 6: äußere PET-Abschirmung
- 7: integrierte innere Abschirmeinrichtung
- 7a: integrierte innere Abschirmeinrichtung
- 7b: integrierte innere Abschirmeinrichtung
- 7c: integrierte innere Abschirmeinrichtung
- 7': integrierte innere Abschirmeinrichtung gemäß Stand der Technik
- 8: Gradientenspulensystem
- 9: Magnetspulenanordnung für statisches Magnetfeld
- 10: Sensorplatine
- 11: Ausleseplatine
- 12a: Durchkontaktierungen im Photosensor
- 12b: Durchkontaktierungen in Sensorplatine
- 12c: Durchkontaktierungen in Ausleseplatine
- 13: Abschirmflächen
- 13a: radial äußere Abschirmflächen
- 13b: radial innere Abschirmflächen
- 14: Abschirmverbindungen, insbesondere Kondensatoren
- 15: Schlitze
- 16: Untersuchungsobjekt
- phi: Umfangsrichtung
- r: radiale Richtung bezogen auf die Längsachse der MRT-Anordnung
- z: Längsachse des Gradientenspulensystems

### Literaturliste

[1] Berneking, Arne, et al. "Design and Characterization of a Gradient-Transparent RF Copper Shield for PET Detector Modules in Hybrid MR-PET Imaging." IEEE Transactions on Nuclear Science 64.5 (2017): 1118-1127.
[2] Berneking, Arne, et al. "RF Coil Performances in Compact Hybrid MR/PET Scanner Design Using an Integrated Shielding", ISMRM 2017
[3] C Pari, A Kolb, A M Schmid, H F Wehrl, J A Disselhorst, P D Soubiran, D Stricker-Shaver and B J Pichler "A novel optically transparent RF shielding for fully integrated PET/MRI systems." Phys. Med. Biol. 62 (2017) 7357-7378
[4] US 9072451 B2
[5] Truhn, Daniel, F. Kiessling, and V. Schulz. "Optimized RF shielding techniques for simultaneous PET/MR." Medical physics 38.7 (2011): 3995-4000.
[6] Salomon, André, et al. "Sparse crystal setting and large axial FOV for integrated whole-body PET/MR." Nuclear Science Symposium and Medical Imaging Conference (NSS/MIC), 2011 IEEE. IEEE, 2011.
[7] US 20060293580 A1
[8] L. Yin, N. Groß-Weege, D. Schug, and V. Schulz "Evaluation of materials for shared-volume PET/MRI inserts", NSS/MIC Conference 2017, Abstract #2531
[9] US20130211233 A
[10] US2014264041A
[11] US 7667457 B2
[12] J. Jin, Elektromagnetic Analysis and Design, Design of RF Shield, CRC Press, 1999
[13] DE 10 2013 108 497 A1
[14] DE 10 2005 015 070 A1
[15] US 2010033186 A1
[16] US 2008265887 A1

## Patentansprüche

1. Hybridbildgebungsvorrichtung umfassend
• eine Magnetresonanztomografie, MRT-Anordnung mit einer RF-Resonatorstruktur (1) und einem Gradientenspulensystem (8) mit einer Längsachse (z),
• eine Emissions-Tomografie, ET-Anordnung mit einer Detektoreinrichtung umfassend einen Photosensor (3) und eine Platinenanordnung mit mindestens einer Ausleseplatine (11), auf der eine ET-Elektronik aufgebracht ist,
• eine innere Abschirmeinrichtung (7, 7a, 7b, 7c) zur gegenseitigen Abschirmung der ET-Elektronik der ET-Anordnung und der RF-Resonatorstruktur (1) der MRT-Anordnung,
wobei der Photosensor (3) bezogen auf die Längsachse radial innerhalb der Platinenanordnung und radial außerhalb der RF-Resonatorstruktur (1) angeordnet ist,
wobei Durchkontaktierungen (12a, 12b, 12c) in der Abschirmeinrichtung (7, 7a, 7b, 7c) vorhanden sind, wobei:
• die innere Abschirmeinrichtung (7a) im Photosensor (3) integriert ist und der Photosensor (3) mit den Durchkontaktierungen (12a) durch die im Photosensor (3) integrierte innere Abschirmeinrichtung (7a) versehen ist, oder
• die innere Abschirmeinrichtung (7b) bezogen auf die Längsachse (z) radial außerhalb des Photosensors (3) in mindestens einer Sensorplatine (10) der Platinenanordnung integriert ist und die mindestens eine Sensorplatine (10) mit den Durchkontaktierungen (12b) für Signal- und Versorgungsleitungen versehen ist, oder
• die innere Abschirmeinrichtung (7c) bezogen auf die Längsachse (z) radial außerhalb des Photosensors (3) in Ausleseplatinen (11) der Platinenanordnung integriert ist, wobei elektrisch leitende Platinenschichten der Ausleseplatinen (11) die innere Abschirmeinrichtung (7c) bilden oder Teil der inneren Abschirmeinrichtung (7c) sind und die Durchkontaktierungen in Sensorplatinen (10) und den Ausleseplatinen (11) vorgesehen sind,
und **dadurch gekennzeichnet, daß** ein RF-Filter in der inneren Abschirmeinrichtung (7, 7a, 7b, 7c) oder der Platinenanordnung integriert ist.

2. Hybridbildgebungsvorrichtung nach Anspruch 1, **dadurch gekennzeichnet, dass** die Emissions-Tomographie-Anordnung eine Positronen Emissions-Tomographie (PET)-Anordnung ist, wobei der Photosensor (3) eine Mehrzahl von kreisförmig angeordneten Sensorelementen umfasst.

3. Hybridbildgebungsvorrichtung nach Anspruch 1 oder 2, **dadurch gekennzeichnet, dass** die innere Abschirmeinrichtung mehrere, sich vorzugsweise überlappende, Abschirmflächen (13, 13a, 13b) aus elektrisch leitfähigem Material umfasst.

4. Hybridbildgebungsvorrichtung nach Anspruch 3, **dadurch gekennzeichnet, dass** die Abschirmflächen (13, 13a, 13b) in mindestens einer Platine (10, 11) der Platinenanordnung integriert oder auf mindestens einer Platine (10, 11) der Platinenanordnung aufgebracht sind, wobei es sich bei der mindestens einen Platine (10, 11) um eine Ausleseplatine (11) und/oder eine Sensorplatine (10) der ET-Anordnung handelt.

5. Hybridbildgebungsvorrichtung nach Anspruch 4, **dadurch gekennzeichnet, dass** die Platinenanordnung mehrlagig ist und zumindest teilweise mehrere Abschirmflächen (13a, 13b) umfasst, wobei die verschiedenen Abschirmflächen (13a, 13b) innerhalb der Platinenanordnung auf mehrere Lagen der Platinenanordnung verteilt sind.

6. Hybridbildgebungsvorrichtung nach einem der Ansprüche 4 oder 5, **dadurch gekennzeichnet, dass** die Abschirmflächen (13, 13a, 13b) innerhalb der Platinenanordnung kapazitiv miteinander verbunden sind.

7. Hybridbildgebungsvorrichtung nach Anspruch 6, **dadurch gekennzeichnet, dass** die kapazitive Verbindung der Abschirmflächen (13a, 13b) innerhalb der Platinenanordnung durch Überlagerung der Abschirmflächen (13a, 13b) innerhalb der Platinenanordnung realisiert ist.

8. Hybridbildgebungsvorrichtung nach einem der vorhergehenden Ansprüche, **dadurch gekennzeichnet, dass** die innere Abschirmeinrichtung eine geschlossene RF-Schirmfläche bildet.

9. Hybridbildgebungsvorrichtung nach einem der vorhergehenden Ansprüche, **dadurch gekennzeichnet, dass** zusätzlich eine äußere Abschirmeinrichtung (6) vorhanden ist, die radial außerhalb der Detektoreinrichtung und der Platinenanordnung angeordnet ist und bevorzugt mit der inneren Abschirmeinrichtung elektrisch verbunden oder elektromagnetisch gekoppelt ist.

10. Hybridbildgebungsvorrichtung nach Anspruch 9, **dadurch gekennzeichnet, dass** Komponenten der ET-Elektronik zwischen innerer Abschirmeinrichtung (7, 7a, 7b, 7c) und äußerer Abschirmeinrichtung (6) angeordnet sind.

11. Hybridbildgebungsvorrichtung nach Anspruch 9 oder 10, **dadurch gekennzeichnet, dass** Signal- und Versorgungsleitungen der ET-Elektronik zwischen innerer Abschirmeinrichtung (7, 7a, 7b, 7c) und äußerer Abschirmeinrichtung (6) angeordnet sind.

12. Hybridbildgebungsvorrichtung nach einem der vorhergehenden Ansprüche, **dadurch gekennzeichnet, dass** es sich bei der Detektoreinrichtung um einen Silizium-Photomultiplier (SiPM) handelt.

## Claims

1. A hybrid imaging apparatus comprising
• a magnetic resonance imaging, MRI arrangement having an RF resonator structure (1) and a gradient coil system (8) with a longitudinal axis (z),
• an emission tomography, ET arrangement having a detector device comprising a photosensor (3) and a circuit board arrangement with at least one readout circuit board (11) on which an ET electronics is arranged,
• an internal shielding device (7, 7a, 7b, 7c) for mutually shielding the ET electronics of the ET arrangement and the RF resonator structure (1) of the MRI arrangement,
wherein the photosensor (3) is arranged, in relation to the longitudinal axis, radially inside the circuit board arrangement and radially outside the RF resonator structure (1),
wherein vias (12a, 12b, 12c) are provided in the shielding device (7, 7a, 7b, 7c), wherein
• the internal shielding device (7a) is integrated in the photosensor (3) and the photosensor is provided with vias (12a) through the internal shielding device (7a) that is integrated in the photosensor (3), or
• the internal shielding device (7b) is integrated, in relation to the longitudinal axis (z), radially outside the photosensor (3) in at least one sensor circuit board (10) of the circuit board arrangement and the at least one sensor circuit board (10) is provided with the vias (12b) for signal and supply lines, or
• the internal shielding device (7c) is integrated, in relation to the longitudinal axis (z), radially outside the photosensor (3) in readout circuit boards (11) of the circuit board arrangement, wherein electrically conducting circuit board layers of the readout circuit boards (11) form the inner shielding device (7c) or are part of the inner shielding device (7c) and the vias are provided in sensor circuit boards (10) and the readout circuit boards (11), and
**characterized in that** an RF filter is integrated in the inner shielding device (7, 7a, 7b, 7c) or the circuit board arrangement.

2. The hybrid imaging apparatus as claimed in claim 1, **characterized in that** the emission tomography arrangement is a positron emission tomography (PET) arrangement, wherein the photosensor (3) comprises a plurality of circularly arranged sensor elements.

3. The hybrid imaging apparatus as claimed in claim 1 or 2, **characterized in that** the internal shielding device comprises a plurality of, preferably overlapping, shielding faces (13, 13a, 13b) made from an electrically conductive material.

4. The hybrid imaging apparatus as claimed in claim 3, **characterized in that** the shielding faces (13, 13a, 13b) are integrated in at least one circuit board (10, 11) of the circuit board arrangement or are arranged on at least one circuit board (10, 11) of the circuit board arrangement, wherein the at least one circuit board (10, 11) is a readout circuit board (11) and/or a sensor circuit board (10) of the ET arrangement.

5. The hybrid imaging apparatus as claimed in claim 4, **characterized in that** the circuit board arrangement is multilayered and comprises at least partially a plurality of shielding faces (13a, 13b), wherein the different shielding faces (13a, 13b) are distributed within the circuit board arrangement over a plurality of layers of the circuit board arrangement.

6. The hybrid imaging apparatus as claimed in one of claims 4 or 5, **characterized in that** the shielding faces (13, 13a, 13b) within the circuit board arrangement are capacitively connected to one another.

7. The hybrid imaging apparatus as claimed in claim 6, **characterized in that** the capacitive connection of the shielding faces (13a, 13b) within the circuit board arrangement is realized by overlay of the shielding faces (13a, 13b) within the circuit board arrangement.

8. The hybrid imaging apparatus as claimed in one of the preceding claims, **characterized in that** the internal shielding device forms a closed RF shield face.

9. The hybrid imaging apparatus as claimed in one of the preceding claims, **characterized in that** additionally an external shielding device (6) is present that is arranged radially outside the detector device and the circuit board arrangement and is preferably electrically connected or electromagnetically coupled to the internal shielding device.

10. The hybrid imaging apparatus as claimed in claim 9, **characterized in that** components of the ET electronics are arranged between the internal shielding device (7, 7a, 7b, 7c) and external shielding device (6).

11. The hybrid imaging apparatus as claimed in claim 9 or 10, **characterized in that** signal and supply lines of the ET electronics are arranged between the internal shielding device (7, 7a, 7b, 7c) and external shielding device (6).

12. The hybrid imaging apparatus as claimed in one of the preceding claims, **characterized in that** the detector device is a silicon photomultiplier (SiPM).

## Revendications

1. Dispositif d'imagerie hybride comprenant :
• un agencement de tomographie par résonance magnétique, TRM, comportant une structure de résonateur RF (1) et un système de bobines à gradient (8) ayant un axe longitudinal (z),
• un agencement de tomographie par émission, TE, comportant un moyen de détection comprenant un photodétecteur (3) et un agencement de circuits imprimés comportant au moins un circuit imprimé de lecture (11) sur lequel est appliquée une électronique de TE,
• un moyen de blindage intérieur (7, 7a, 7b, 7c) pour le blindage mutuel de l'électronique de TE de l'agencement de TE et de la structure de résonateur RF (1) de l'agencement de TRM,
le photodétecteur (3) étant disposé, par rapport à l'axe longitudinal, radialement à l'intérieur de l'agencement de circuits imprimés et radialement à l'extérieur de la structure de résonateur RF (1), des vias (12a, 12b, 12c) étant présents dans le moyen de blindage (7, 7a, 7b, 7c), dans lequel :
• le moyen de blindage intérieur (7a) est intégré dans le photodétecteur (3) et le photodétecteur (3) est pourvu des vias (12a) à travers le moyen de blindage intérieur (7a) intégré dans le photodétecteur (3), ou
• le moyen de blindage intérieur (7b) est intégré, par rapport à l'axe longitudinal (z), radialement à l'extérieur du photodétecteur (3) dans au moins un circuit imprimé de détection (10) de l'agencement de circuits imprimés et ledit au moins un circuit imprimé de détecteur (10) est pourvu des vias (12b) pour des lignes de signal et d'alimentation, ou
• le moyen de blindage intérieur (7c) est intégré, par rapport à l'axe longitudinal (z), radialement à l'extérieur du photodétecteur (3), dans des circuits imprimés de lecture (11) de l'agencement de circuits imprimés, des couches de circuit imprimé électriquement conductrices des circuits imprimés de lecture (11) formant le moyen de blindage intérieur (7c) ou faisant partie du moyen de blindage intérieur (7c) et les vias étant prévus dans des circuits imprimés de détection (10) et les circuits imprimés de lecture (11),
et **caractérisé en ce qu'**un filtre RF est intégré dans le moyen de blindage intérieur (7, 7a, 7b, 7c) ou l'agencement de circuits imprimés.

2. Dispositif d'imagerie hybride selon la revendication 1, **caractérisé en ce que** l'agencement de tomographie par émission est un agencement de tomographie par émission de positrons (TEP), dans lequel le photodétecteur (3) comprend une pluralité d'éléments de détection disposés de manière circulaire.

3. Dispositif d'imagerie hybride selon la revendication 1 ou 2, **caractérisé en ce que** le moyen de blindage intérieur comprend plusieurs surfaces de blindage (13, 13a, 13b) en matériau électriquement conducteur, de préférence se chevauchant.

4. Dispositif d'imagerie hybride selon la revendication 3, **caractérisé en ce que** les surfaces de blindage (13, 13a, 13b) sont intégrées dans au moins un circuit imprimé (10, 11) de l'agencement de circuits imprimés ou sont appliquées sur au moins un circuit imprimé (10, 11) de l'agencement de circuits imprimés, ledit au moins un circuit imprimé (10, 11) étant un circuit imprimé de lecture (11) et/ou un circuit imprimé de détection (10) de l'agencement de TE.

5. Dispositif d'imagerie hybride selon la revendication 4, **caractérisé en ce que** l'agencement de circuits imprimés est multicouche et comprend au moins partiellement plusieurs surfaces de blindage (13a, 13b), les différentes surfaces de blindage (13a, 13b) étant réparties à l'intérieur de l'agencement de circuits imprimés sur plusieurs couches de l'agencement de circuits imprimés.

6. Dispositif d'imagerie hybride selon l'une des revendications 4 ou 5, **caractérisé en ce que** les surfaces de blindage (13, 13a, 13b) sont reliées entre elles de manière capacitive à l'intérieur de l'agencement de circuits imprimés.

7. Dispositif d'imagerie hybride selon la revendication 6, **caractérisé en ce que** la liaison capacitive des surfaces de blindage (13a, 13b) à l'intérieur de l'agencement de circuits imprimés est réalisée par superposition des surfaces de blindage (13a, 13b) à l'intérieur de l'agencement de circuits imprimés.

8. Dispositif d'imagerie hybride selon l'une des revendications précédentes, **caractérisé en ce que** le moyen de blindage intérieur forme une surface de blindage RF fermée.

9. Dispositif d'imagerie hybride selon l'une des revendications précédentes, **caractérisé en ce qu'**un moyen de blindage extérieur (6) est en outre présent, qui est disposé radialement à l'extérieur du moyen de détection et de l'agencement de circuits imprimés et qui est de préférence relié électriquement ou couplé électromagnétiquement au moyen de blindage intérieur.

10. Dispositif d'imagerie hybride selon la revendication 9, **caractérisé en ce que** des composants de l'électronique de TE sont disposés entre le moyen de blindage intérieur (7, 7a, 7b, 7c) et le moyen de blindage extérieur (6).

11. Dispositif d'imagerie hybride selon la revendication 9 ou 10, **caractérisé en ce que** les lignes de signal et d'alimentation de l'électronique de TE sont disposées entre le moyen de blindage intérieur (7, 7a, 7b, 7c) et le moyen de blindage extérieur (6).

12. Dispositif d'imagerie hybride selon l'une des revendications précédentes, **caractérisé en ce que** le moyen de détection est un photomultiplicateur au silicium (SiPM).
